# EUROPÄISCHE PATENTSCHRIFT

(11) **EP 1 744 706 B1**
(45) Veröffentlichungstag und Bekanntmachung des Hinweises auf die Patenterteilung: **05.05.2010**
(21) Anmeldenummer: 05700531.6
(22) Anmeldetag: 11.01.2005
(51) Int. Cl.: A61F 2/90

(54) **STENT**
STENT
STENT

(30) Priorität: 03.05.2004 DE 102004022044
(43) Veröffentlichungstag der Anmeldung: 24.01.2007
(73) Patentinhaber: Qualimed Innovative Medizinprodukte GmbH, 21423 Winsen (DE)
(72) Erfinder: NISSL, Thomas, 21441 Garstedt (DE)
(74) Vertreter: Ksoll, Peter
(86) Internationale Anmeldenummer: PCT/DE2005/000018
(87) Internationale Veröffentlichungsnummer: WO 2005/104990

(56) Entgegenhaltungen:
- EP-A- 1 088 528
- WO-A-02/24111
- WO-A-98/22159
- WO-A-99/16387
- WO-A-2004/084769

## Beschreibung

Die Erfindung betrifft einen Stent gemäß den Merkmalen im Oberbegriff von Patentanspruch 1.

Stents dienen zur permanenten oder auch nur zeitweisen Schienung von Körperröhren, die infolge einer Stenose verschlossen oder verengt sind.

Die Stents weisen ein tubuläres Stützgerüst aus Metall auf, welches aus mehreren Ringsegmenten besteht. Diese sind aus sich über Übergangsabschnitte endlos aneinander schließenden Segmentstreben gebildet. In Längsachse des Stents benachbarte Ringsegmente sind durch Verbinderstreben gekoppelt.

Stents gibt es in verschiedenartigsten Ausführungsformen und Designs des Stützgerüsts. Die WO 96/26689, die US-A-5,861,027, die DE 297 02 671 U1 oder die DE 295 21 206 U1 werden als exemplarische Beispiele genannt. Ein Stent, der die Merkmale des Oberbegriffs des Anspruchs 1 aufweist, is aus der WO 99/16387 bekannt.

Die Stents werden durch Kathetertechniken und ähnliche Einführhilfen in das intrakorporale Gefäß im Bereich der Stenose eingebracht und dort abgesetzt, wobei das Stützgerüst vom Ausgangszustand in einen demgegenüber im Durchmesser vergrößerten Stützzustand aufweitbar ist. Dieser Aufweitvorgang kann selbsttätig bei sogenannten selbst expandierenden Stents erfolgen, er kann aber auch mit Hilfe eines geeigneten Werkzeugs, beispielsweise eines Ballonkatheters herbeigeführt werden. Im Gefäß fungieren die Stents als Gefäßprothese zur Abstützung der Gefäßinnenwände.

Zu den selbstexpandierenden Stents zählen solche aus sogenannten Formgedächtnislegierungen. Eine Formgedächtnislegierung ist beispielsweise Nitinol, bei der es sich um eine Nickel-Titan-Legierung handelt. Diese weist zwei abgrenzbare Zustände auf, die temperaturabhängig eintreten. Nach Vorbehandlung ist Nitinol im kalten Zustand martensitisch, d.h. plastisch verformbar ohne eine relevante elastische Rückstellkraft. Bei Erwärmung geht das Material in einen austhenitischen elastischen Zustand über. Diese Formgedächtniseigenschaft wird zur Selbstentfaltung des Stents ausgenutzt.

Ein gängiges Herstellungsverfahren zur Fertigung von Stents besteht darin, ein dünnwandiges metallisches Rohr entsprechend der Kontur des Stützgerüstes zu schlitzen und anschließend aufzuweiten. Der Schlitzvorgang erfolgt mittels Laserschneiden. Hierbei kommen üblicherweise Rohre mit einem Ausgangsdurchmesser von 1,4 - 1,8 mm zum Einsatz, um hieraus Stents mit einem Durchmesser von 5 - 12 mm zu erzeugen. Bei den bislang üblichen Designs des Stützgerüstes ist in Folge der Schneidtechnologie eine parallele Schnittgeometrie der Segmentstreben vorgesehen und nicht anders möglich. Dies führt jedoch zu hohen Kerbspannungen an den Enden der Segmentstreben bzw. im Bereich der Übergangsabschnitte und damit zu einer hohen Bruchgefahr.

Der Erfindung liegt daher ausgehend vom Stand der Technik die Aufgabe zugrunde, einen hinsichtlich des Spannungsverlaufs in den Segmentstreben verbesserten Stent zu schaffen, bei dem die Spannungen in den Enden der Segmentstreben reduziert und über die Länge der Segmentstreben verteilt sind.

Die Lösung dieser Aufgabe besteht nach der Erfindung in einem Stent gemäß den Merkmalen von Patentanspruch 1.

Der erfindungsgemäße Stent weist ein tubuläres Stützgerüst auf, welches von einem Ausgangszustand in einen Stützzustand aufweitbar ist. Das Stützgerüst besteht aus in Stentlängsachse aufeinander folgenden Ringsegmenten, die aus sich in Umfangsrichtung des Stützgerüstes endlos aneinander schließenden Segmentstreben gebildet sind. Benachbarte Ringsegmente sind durch Verbinderstreben gekoppelt. Kernpunkt der Erfindung bildet die Maßnahme, dass die Segmentstreben wellenförmig gekrümmt sind und sich die quer zur Strebenlängsachse gemessene Breite der Segmentstreben vom mittleren Bereich ausgehend in Richtung zu den Übergangsabschnitten hin vergrößert. Die in Umfangsrichtung des Stützgerüstes gemessene Breite der Segmentstreben bleibt über deren Längenverlauf hin gleich. Die Segmentstreben sind folglich lotrecht gemessen im mittleren Bereich schmäler als an ihren Enden, wodurch die Spannungen über die gesamte Länge einer Segmentstrebe verteilt und die höher belasteten Enden durch die größere Schnittbreite entlastet werden. Dies führt zu einer verminderten Bruchgefahr in den besonders kritischen Bereichen an den Enden der Segmentstreben und damit zu einer deutlich gesteigerten Lebensdauer eines Stent.

Das Stützgerüst weist ein Wellendesign auf ohne parallel zueinander verlaufenden gradlinige Strebenabschnitte, wobei sich die Segmentstreben einem kontinuierlichen Konturverlauf folgend vom mittleren Bereich jeweils zu ihren Enden hin verbreitern.

Vorzugsweise ändert sich das Verhältnis des Wellenradius zur Breite einer Segmentstrebe von der Mitte ausgehend zu den Enden der Segmentstrebe hin in einem Verhältnis von 10:1 bis 15:1.

Der erfindungsgemäße Stent weist bei hoher Flexibilität eine sehr gute Krimpbarkeit auf. Im Stützzustand zeichnet er sich durch seine hohe Stabilität und hohe Radialsteifigkeit bei besserer Restenoserate aus.

Der Stent ist aus Metall gefertigt. Hierbei können alle verformbaren medizinisch möglichen Metalle bzw. Metalllegierungen zum Einsatz gelangen, z.B. Edelstahl, Kobaltlegierungen (Phynox), Reineisen oder insbesondere Nickel-Titan-Legierungen.

Die Herstellung des Stützgerüstes erfolgt durch Schlitzen einer vorzugsweise metallischen Röhre mittels eines Laserstrahls. Dieser fährt bei einer Fokuseinstellung mit einer Breite von beispielsweise von 20 - 30 µm die vorgegebene Schnittkontur ab. Die Geometrieänderung in der Breite der Segmentstreben erfolgt hierbei durch die entsprechende Radienwahl und Radienänderung in Längsrichtung einer Segmentstrebe zum Übergangsabschnitt hin.

Von besonderem Vorteil ist, dass die Kontur bzw. Konfiguration des Stützgerüstes durch einfaches Schlitzen der Ausgangsröhre erzeugt werden kann, ohne dass die Geometrie aus dem Vollen herausgearbeitet werden muss.

Für die Praxis interessant kann der erfindungsgemäße Stent auch als Kunststoff-Stent sein. Hierbei ist insbesondere die Verwendung von bioresorbierbaren Kunststoffen geplant. Vorzugsweise wird der Stent dann als Spritzformteil ausgeführt.

Durch die erfindungsgemäße Formgebung der Segmentstreben bzw. des Stützgerüstes kann auch hier der Spannungsverlauf optimiert werden.

Ferner sind 1. Verbinderstreben und 2. Verbinderstreben vorgesehen. Jede Verbinderstrebe weist einen in Umfangsrichtung des Stützgerüsts verlaufenden Schenkel auf, der beidseitig über Axialabschnitte an einen Übergangsabschnitt angeschlossen ist. Diese Konfiguration der Verbinderstreben trägt zur Längenstabilität eines Stents bei.

Eine sich theoretisch durch einen Aufweitvorgang und den Übergang der Segmentstreben in eine aufgeweitete gestreckte Form ergebende Längenverkürzung des Stützgerüsts wird durch den Schenkel in den Verbinderstreben ausgeglichen.

Die Axialabschnitte der 1. Verbinderstreben sind ebenfalls wellenförmig gekrümmt. Hierbei nimmt die Breite der Axialabschnitte quer zur Längsachse der Axialabschnitte gemessen von den Schenkeln ausgehend in Richtung zu den Übergangsabschnitten zu.

Als besonders vorteilhaft wird ein Verhältnis des Krümmungs- bzw. Wellenradius der Axialabschnitte zur Breite der Axialabschnitte angesehen, welches sich von den Schenkeln ausgehend zu den Enden hin in einem Bereich zwischen 12:1 bis 20:1 ändert.

Die in Umfangsrichtung verlaufenden Schenkel der Verbindungsstreben sind gemäß den Merkmalen von Patentanspruch 2 in dem Raum zwischen zwei mit axialem Abstand benachbarten Ringsegmenten angeordnet.

Nach den Merkmalen von Patentanspruch 3 erstrecken sich die 1. Verbinderstreben jeweils von dem Tiefsten zweier aneinander geschlossener Segmentstreben eines Ringsegments in das Tiefste von zwei aneinander geschlossenen Segmentstreben des benachbarten Ringsegments (Patentanspruch 4).

Demgegenüber erstrecken sich die 2. Verbinderstreben jeweils von der Spitze zweier aneinander geschlossener Segmentstreben eines Ringsegments bis zur Spitze zweier aneinander geschlossener Segmentstreben des benachbarten Ringsegments.

In diesem Zusammenhang in gemäß dem Merkmal von Patentanspruch 5 vorgesehen, dass die 1. Verbinderstreben eines Ringsegments und die 2. Verbinderstreben des benachbarten Ringsegments in Umfangsrichtung versetzt angeordnet sind.

Eine die Anwendung des erfindungsgemäßen Stents verbessernde Maßnahme besteht nach den Merkmalen von Patentanspruch 6 darin, dass jeder dritte stirnseitige Übergangsabschnitt an den in Stentlängsachse gesehen endseitigen Ringsegmenten ein gegenüber den benachbarten Übergangsabschnitten axial vorstehendes verbreitertes Kopfende aufweist.

Die vorteilhafterweise gerundeten Kopfenden gewährleisten einen schonenden Kontakt der stirnseitigen Enden des Stents an einer Gefäßwand. Hierdurch werden die Gefäßwände sowohl beim Setzen als auch beim Entfernen eines Stents weniger traumatisiert. Im gekrimpten Zuständen decken die Kopfenden die benachbarten Übergangsabschnitte ab. Die Gefahr einer Verletzung der umliegenden Gefäßwände wird hierdurch deutlich verringert.

Insgesamt ergibt sich ein Stützgerüst mit hoher Radialsteifigkeit im Stützzustand. Dies gewährleistet eine sehr gute und homogene Schienung der Gefäßwand mit einer funktional zweckmäßigen Abstützung. Durch die erfindungsgemäße Ausgestaltung der Segmentstreben können unzulässig hohe Kerbspannungen vermieden werden. Demzufolge kann der erfindungsgemäße Stent sehr gut gekrimmt und aufgeweitet werden. Der Stent kann beispielsweise auf einem Ballonkatheter angeordnet sehr gut durch die Windungen eines Körpergefäßes gebracht werden. Diese Leichtgängigkeit bewirkt sowohl für den Anwender als auch für den Patienten eine hohe Sicherheit bei der Implantation.

Die Erfindung ist nachfolgend anhand eines in den Zeichnungen dargestellten Ausführungsbeispiels näher beschrieben. Es zeigen:
- Figur 1: einen Ausschnitt aus dem Stentmuster eines erfindungsgemäßen Stents im Ausgangszustand in einer Abwicklung;
- Figur 2: einen endseitigen Ausschnitt des Stents im Stützzustand;
- Figur 3: ein erstes Modell einer Segmentstrebe;
- Figur 4: ein zweites Modell einer Segmentstrebe;
- Figur 5: ein Diagramm mit der Darstellung der Breite einer Segmentstrebe im Verhältnis zum Wellenradius und
- Figur 6: ein Diagramm mit der Darstellung der Breite eines Verbinders im Verhältnis zum Wellenradius.

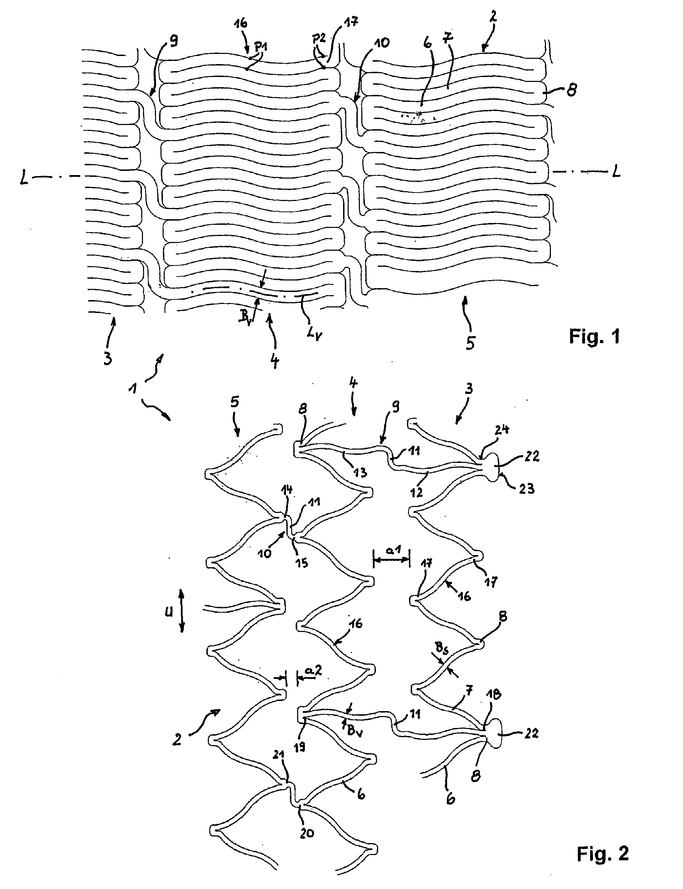

Die Figuren 1 und 2 zeigen jeweils einen Ausschnitt aus dem Stentmuster eines erfindungsgemäßen Stents 1 in einer Abwicklung. Die Figur 1 zeigt die Abwicklung des Stents 1 im unaufgeweiteten Ausgangszustand. Figur 2 gibt die Abwicklung des Stentmusters im aufgeweiteten Stützzustand wieder.

Der Stent 1 ist aus Metall, insbesondere aus Nitinol, gefertigt. Er besitzt ein tubuläres Stützgerüst 2 aus mehreren hintereinander folgenden Ringsegmenten 3, 4, 5. Die Länge eines Stents 1 kann grundsätzlich unterschiedlich sein. Die Figuren 1 und 2 geben nicht die gesamte Anzahl der Ringsegmente 3, 4, 5 des Stents 1 wieder.

Die Ringsegmente 3, 4, 5 werden von Segmentstreben 6, 7 gebildet, die sich über Übergangsabschnitte 8 endlos aneinander schließen. Untereinander sind die Ringsegmente 3, 4, 5 durch Verbinderstreben 9, 10 gekoppelt. Man erkennt 1. lange Verbinderstreben 9 und 2. kurze Verbinderstreben 10. In jeder Verbinderstrebe 9, 10 ist ein in Umfangsrichtung U des Stützgerüstes 2 verlaufender Schenkel 11 vorgesehen, der beidseitig über Axialabschnitte 12, 13; 14, 15 an einen Übergangsabschnitt 8 angeschlossen ist. Hierbei sind die Schenkel 11 jeweils in dem Raum zwischen zwei mit axialem Abstand a1, a2 benachbarten Ringsegmenten 3, 4 bzw. 4, 5 angeordnet. Es wird deutlich, dass die Abstände a1 und a2 zwischen den Ringsegmenten 3 und 4 bzw. den Ringsegmenten 4 und 5 im aufgeweiteten Stützzustand des Stents 1, wie in Figur 2 dargestellt, unterschiedlich groß bemessen sind, wobei a1 größer ist als a2.

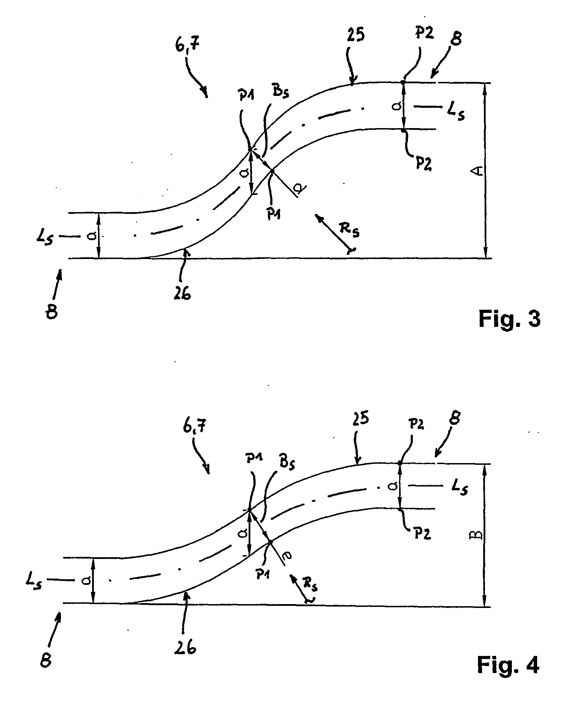

Die Segmentstreben 6, 7 sind wellenförmig gekrümmt (siehe hierzu auch Figuren 3 und 4). Hierbei vergrößert sich die quer zur Strebenlängsachse Lₛ lotrecht gemessene Breite B_{S} jeder Segmentstrebe 6, 7 vom mittleren Bereich 16 ausgehend in Richtung zu den Übergangsabschnitten 8. Die Segmentstreben 6, 7 sind im mittleren Bereich 16 zwischen den Punkten P1 schmaler als an ihren Enden 17 zwischen den Punkten P2. Der Radius Rₛ ändert sich über dem Längenverlauf einer Segmentstrecke 6, 7 und nimmt vom mittleren Bereich 16 ausgehend in Richtung zu den Übergangsabschnitten 8 hin zu. Hierdurch wird eine durch äußere Belastung im Stützzustand in einer Körperröhre in den Segmentstreben 6, 7 erzeugte innere Spannung über die gesamte Länge einer Segmentstrebe 6, 7 verteilt. Die höher belasteten Enden 17 werden durch die dort größere Breite B_{S} entlastet. Auf diese Weise wird die Bruchgefahr in den kritischen Bereichen am Übergang zwischen den Enden 17 der Segmentstreben 6, 7 zu den Übergangsbereichen 8 vermindert.

Die Axialabschnitte 12, 13 der 1. Verbinderstreben 9 weisen eine zur Kontur der Segmentstreben 8, 7 konforme wellenförmige Krümmung auf. Die quer zur Längsachse Lᵥ der Axialabschnitte 12, 13 gemessene Breite B_{V} der Axialabschnitte 12 bzw. 13 nimmt vom Schenkel 11 ausgehend in Richtung zu den Übergangsabschnitten 8 hin zu.

Die 1. Verbinderstreben 9 erstrecken sich mit ihren Axialabschnitten 12, 13 jeweils vom Tiefsten 18 zweier aneinander geschlossener Segmentstreben 6, 7 eines Ringsegments 3 in das Tiefste 19 zweier aneinander geschlossener Segmentstreben 6, 7 eines benachbarten Ringsegments 4. Demgegenüber verlaufen die 2. Verbinderstreben 10 jeweils von der Spitze 20 zweier aneinander angeschlossener Segmentstreben 6, 7 eines Ringsegments 4 bis zur Spitze 21 zweier aneinander geschlossener Segmentstreben 6, 7 eines benachbarten Ringsegments 5. Die 1. Verbinderstreben 9 und die 2. Verbinderstreben 10 sind hierbei von Ringsegment 3, 4 zu Ringsegment 4, 5 jeweils in Umfangsrichtung U versetzt zueinander angeordnet.

Wie anhand der Figur 2 zu erkennen, weist jeder dritte stirnseitige Übergangsabschnitt 8 an den endseitigen Ringsegmenten 3 ein gegenüber den benachbarten Übergangsabschnitten 8 axial vorstehendes verbreitertes Kopfende 22 auf. Jedes Kopfende 22 besitzt einen konvex gerundeten Stirnabschnitt 23 und konkav gerundete Kehlabschnitte 24 zu den Übergangsabschnitten 8 hin. Im gekrimpten Zustand übergreifen die Kopfenden 22 mit ihren Kehlabschnitten 24 die benachbarten Übergangsabschnitte 8 und decken diese ab. Die benachbarten Übergangsabschnitte 8 liegen so geschützt bzw. verdeckt durch die Kopfenden 22. Hierdurch werden die Gefäßwände sowohl beim Setzen als auch beim Entfernen eines Stents 1 weniger traumatisiert. Zudem gewährleisten die gerundeten Kopfenden 22 eine behutsame Anlage des Stents 1 an der Gefäßwand beim Setzen.

Die Figuren 3 und 4 zeigen zwei Modelle einer Segmentstrebe 6 bzw. 7 mit der Darstellung des Breitenverlaufs über die Länge der Segmentstrebe 6, 7 gesehen.

Die Breite a am Anfang und Ende einer Segmentstrebe 6, 7 ist gleich. Die Breite a in Umfangsrichtung U ist über die gesamte Länge der Segmentstrebe 6, 7 ebenfalls gleich "a". Die quer zur Strebenlängsachse Lₛ gemessene Breite Bₛ nimmt vom mittleren Bereich 16 ausgehend in Richtung zu den Übergangsabschnitten 8 hin zu. Die Breitenzunahme ist abhängig von der Steigung und dem Wellenradius Rₛ einer Segmentstrebe 6 bzw. 7. Die Übergänge 25, 26 zu den Übergangsabschnitten 8 hin sind ebenfalls als Radien ausgeformt. Daher gibt es hier einen kontinuierlichen Übergang von der Segmentbreite a zur lotrecht gemessenen Segmentbreite Bₛ im mittleren Bereich 16. Dieser Konturverlauf führt beim Aufdehnen eines Stents 1 zu einer kontinuierlichen Verformung.

Durch Vergleich der in den Figuren 3 und 4 dargestellten Modelle wird deutlich, dass durch eine Anpassung der Abstände A und B zwischen Segmentanfang und Segmentende die Segmentbreite sehr gut eingestellt werden kann. Wird der Abstand A größer als der Abstand B gewählt, folgt hieraus, dass die lotrechte Breite d kleiner ist als die lotrechte Breite e (A>B ⇒ d<e).

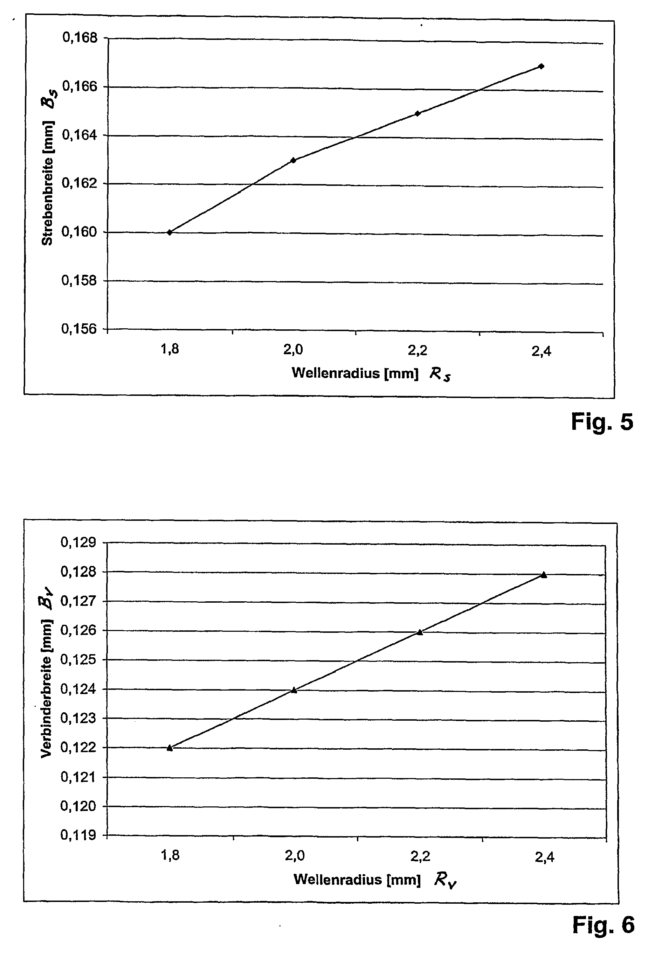

Die Figur 5 zeigt ein Liniendiagramm, in dem die Breite B_{S} aufgetragen ist über den Wellenradius R_{S} einer Segmentstrebe 6, 7. Dargestellt ist ein Ausschnitt aus der Länge einer Segmentstrebe 6, 7. Die Strebenbreite B_{S} beträgt im mittleren Bereich 0,16 mm bei einem Radius R_{S} von 1,8 mm. Die Strebenbreite B_{S} nimmt hierbei annähernd gleichmäßig zum Ende hin zu. Man erkennt, dass der Wellenradius R_{S} bei der Strebenbreite B_{S} von von 0,167 mm bei 2,4 mm liegt. Im konkret ausgemessenen Ausführungsbeispiel beträgt die Strebenbreite B_{S} am Ende einer Segmentstrebe 0,175 mm. Insgesamt sollte das Verhältnis von Wellenradius R_{S} zu Strebenbreite B_{S} in einem Bereich zwischen 10:1 bis auf 15:1 ansteigen.

Aus der Figur 6 geht ein Liniendiagramm hervor, bei dem die Breite B_{V} einer Verbinderstrebe 9 bzw. eines Axialabschnitts 12, 13 über den Wellenradius R_{W} aufgetragen ist. Das Verhältnis von Wellenradius R_{W} zur Verbinderbreite B_{V} nimmt linear zu. Hierbei steigt das Verhältnis der Verbinderbreite B_{V} von der Mitte ausgehend in Richtung zu den Übergangsabschnitten 8 an, und zwar in einem Verhältnis von 12:1 bis 20:1.

### Bezugszeichenaufstellung

- 1 -: Stent
- 2 -: Stützgerüst
- 3 -: Ringsegment
- 4 -: Ringsegment
- 5 -: Ringsegment
- 6 -: Segmentstrebe
- 7 -: Segmentstrebe
- 8 -: Übergangsabschnitt
- 9 -: Verbinderstrebe
- 10 -: Verbinderstrebe
- 11 -: Schenkel
- 12 -: Axialabschnitt
- 13 -: Axialabschnitt
- 14 -: Axialabschnitt
- 15 -: Axialabschnitt
- 16 -: mittlerer Bereich v. 6, 7
- 17 -: Ende v. 6, 7
- 18 -: Tiefstes
- 19 -: Tiefstes
- 20 -: Spitze
- 21 -: Spitze
- 22 -: Kopfende
- 23 -: Stirnabschnitt
- 24 -: Kehlabschnitt
- 25 -: Übergang
- 26 -: Übergang
- a1 -: Abstand
- a2 -: Abstand
- B_{S} -: Breite v. 6, 7
- B_{V} -: Breite v. 12, 13
- L -: Stentlängsachse
- Lₛ -: Längsachse v. 6, 7
- Lᵥ -: Längsachse v. 12, 13
- P1 -: Punkt
- P2 -: Punkt
- R_{S} -: Radius v. 6, 7
- R_{W} -: Radius v. 12, 13
- U -: Umfangsrichtung
- A -: Abstand
- B -: Abstand
- a -: Breite
- d -: Breite
- e -: Breite

## Patentansprüche

1. Stent zum Anordnen in einer Körperröhre mit einem tubulären Stützgerüst (2) aus axial aufeinander folgenden Ringsegmenten (3-5), die aus sich über Übergangsabschnitte (8) endlos aneinander schließenden Segmentstreben (6, 7) gebildet und benachbarte Ringsegmente (3-5) durch Verbinderstreben (9, 10) gekoppelt sind, wobei die quer zur Strebenlängsachse (L_{S}) gemessene Breite (B_{S}) jeder Segmentstrebe (6, 7) sich von ihrem mittleren Bereich (16) ausgehend in Richtung zu den Übergangsabschnitten (8) hin vergrößert, **dadurch gekennzeichnet, dass** die Segmentstreben (6, 7) wellenförmig gekrümmt sind und dass 1. Verbinderstreben (9) sowie 2. Verbinderstreben (10) vorgesehen sind, wobei jede Verbinderstrebe (9, 10) einen in Umfangsrichtung des Stützgerüstes (2) verlaufenden Schenkel (11) aufweist, der beidseitig über Axialabschnitte (12, 13 bzw. 14, 15) an einen Übergangsabschnitt (8) angeschlossen ist, und die Axialabschnitte (12, 13) der 1. Verbinderstreben (9) wellenförmig gekrümmt sind und sich die quer zur Längsachse (L_{V}) der Axialabschnitte (12, 13) gemessene Breite (B_{V}) der Axialabschnitte (12, 13) vom Schenkel (11) ausgehend in Richtung zu den Übergangsabschnitten (8) hin vergrößert.

2. Stent nach Anspruch 1, **dadurch gekennzeichnet, dass** die Schenkel (11) zwischen zwei mit axialem Abstand (a) benachbarten Ringsegmenten (3, 4 bzw. 4, 5) angeordnet sind.

3. Stent nach Anspruch 1 oder 2, **dadurch gekennzeichnet, dass** die 1. Verbinderstreben (9) sich jeweils von dem Tiefsten (18) zweier aneinander geschlossener Segmentstreben (6, 7) eines Ringsegments (3) in das Tiefste (19) zweier aneinander geschlossener Segmentstreben (6, 7) eines benachbarten Ringsegments (4) erstrecken.

4. Stent nach einem der Ansprüche 1 bis 3, **dadurch gekennzeichnet, dass** die 2. Verbinderstreben (10) sich jeweils von der Spitze (20) zweier aneinander geschlossener Segmentstreben (6, 7) eines Ringsegments (4) bis zur Spitze (21) zweier aneinander geschlossener Segmentstreben (6, 7) eines benachbarten Ringsegments (5) erstrecken.

5. Stent einem der Ansprüche 1 bis 4, **dadurch gekennzeichnet, dass** die 1. Verbinderstreben (9) und die 2. Verbinderstreben (10) zwischen den Ringsegmenten (3, 4 bzw. 4, 5) in Umfangsrichtung (U) versetzt angeordnet sind.

6. Stent einem der Ansprüche 1 bis 5, **dadurch gekennzeichnet, dass** jeder dritte stirnseitige Übergangsabschnitt (8) an den endseitigen Ringsegmenten (3) ein gegenüber den benachbarten Übergangsabschnitten (8) axial vorstehendes verbreitertes Kopfende (22) aufweist.

## Claims

1. Stent for placement in a body vessel, with a tubular support frame (2) composed of axially sequential ring segments (3-5) which are formed by segmented braces (6, 7) which are interconnected continuously in via transfer sections (8) and adjacent ring segments (3-5) are coupled by connecting braces (9, 10), wherein the width (Bₛ) of each segmented brace (6, 7) measured transversely to the longitudinal axis of each brace (Lₛ) increases from its central zone (16) in the direction of the transfer sections (8), **characterised in that** the segmented braces (6, 7) are curved in a wavelike manner and that first connecting braces (9) and second connecting braces (10) are provided, wherein each connecting brace (9, 10) has a limb (11) running in the circumferential direction of the support frame (2), which limb is attached on both sides via axial sections (12, 13 and 14, 15) to a transfer section (8), and the axial sections (12, 13) of the first connecting braces (9) are curved in a wavelike manner and the width (Bᵥ) of the axial sections (12, 13) measured transversely to the longitudinal axis (Lᵥ) of the axial sections (12, 13) increases from the limb (11) in the direction of the transfer sections (8).

2. Stent according to claim 1, **characterised in that** the limbs (11) are arranged between two adjacent ring segments (3, 4 and 4, 5) with axial clearance (a).

3. Stent according to claim 1 or 2, **characterised in that** the first connecting braces (9) each extend from the base (18) of two interconnected segmented braces (6, 7) of one ring segment (3) into the base (19) of two interconnected segmented braces (6, 7) of an adjacent ring segment (4).

4. Stent according to one of claims 1 to 3, **characterised in that** the second connecting braces (10) each extend from the tip (20) of two interconnected segmented braces (6, 7) of one ring segment (4) to the tip (21) of two interconnected segmented braces (6, 7) of an adjacent ring segment (5).

5. Stent according to one of claims 1 to 4, **characterised in that** the first connecting braces (9) and the second connecting braces (10) are arranged offset in the circumferential direction (U) between the ring segments (3, 4 and 4, 5).

6. Stent according to one of claims 1 to 5, **characterised in that** every third front end transfer section (8) has a widened head end (22) on the back end ring segments (3) which projects axially beyond the adjacent transfer sections (8).

## Revendications

1. Stent à disposer dans un tube corporel comprenant une structure d'appui tubulaire (2) constituée de segments circulaires se suivant axialement (3-5) qui sont formés de tiges de segments (6, 7) adjointes les unes aux autres en continu par le biais de segments de transition (8) et des segments circulaires voisins (3-5) sont couplés par des tiges de liaison (9, 10), dans lequel la largeur mesurée (B_{S}) perpendiculairement à l'axe longitudinal des tiges (L_{S}) de chaque tige de segment (6, 7) augmente depuis sa zone médiane (16) dans le sens des segments de transition (8), **caractérisé en ce que** les tiges de segments (6, 7) sont courbées de façon ondulée et que des premières tiges de liaison (9) et des deuxièmes tiges de liaison (10) sont prévues, chaque tige de liaison (9, 10) présentant une branche (11) évoluant dans le sens périphérique de la structure d'appui (2) qui est adjointe des deux côtés à un segment de transition (8) par le biais de segments axiaux (12, 13 ou 14, 15) et les segments axiaux (12, 13) de la première tige de liaison (9) étant courbés de façon ondulée et la largeur mesurée (B_{V}) des segments axiaux (12, 13) perpendiculairement à l'axe longitudinal (L_{V}) des segments axiaux (12, 13) augmentant depuis la branche dans le sens des segments de transition (8).

2. Stent selon la revendication 1, **caractérisé en ce que** la branche (11) est disposée entre deux segments circulaires (3, 4 ou 4, 5) voisins présentant un intervalle axial (a).

3. Stent selon la revendication 1 ou 2, **caractérisé en ce que** la première tige de liaison (9) s'étend respectivement de la zone la plus profonde (18) de deux tiges de segments adjointes (6, 7) d'un segment circulaire (3) dans la zone la plus profonde (19) de deux tiges de segments adjointes (6, 7) d'un segment circulaire voisin (4).

4. Stent selon l'une des revendications 1 à 3, **caractérisé en ce que** les deuxièmes tiges de liaison (10) s'étendent respectivement de la pointe (20) de deux tiges de segments adjointes (6, 7) d'un segment circulaire (4) jusqu'à la pointe (21) de deux tiges de segment adjointes (6, 7) d'un segment circulaire voisin (5).

5. Stent selon l'une des revendications 1 à 4, **caractérisé en ce que** la première tige de liaison (9) et la deuxième tige de liaison (10) sont disposées de façon décalée entre les segments circulaires (3, 4 ou 4, 5) dans le sens périphérique (U).

6. Stent selon l'une des revendications 1 à 5, **caractérisé en ce qu'**un segment de transition (8) sur trois, de face frontale, présente sur les segments circulaires terminaux (3), une tête élargie (22) dépassant axialement faisant face aux segments de transition voisins (8)
